# EUROPÄISCHE PATENTANMELDUNG

(11) **EP 4 717 214 A1**
(43) Veröffentlichungstag der Anmeldung: **01.04.2026**
(21) Anmeldenummer: 24203277.9
(22) Anmeldetag: 27.09.2024
(51) Int. Cl.: A61B 18/14, A61B 18/00

(54) **GEWEBEVERSIEGELUNGSINSTRUMENT**

(71) Anmelder: Erbe Elektromedizin GmbH, 72072 Tübingen (DE)
(72) Erfinder: MAYER, Volker, 72072 Tuebingen (DE); WEILER, Rolf, 72074 Tuebingen (DE); HEIDER, Tanja, 72138 Kirchentellinsfurt (DE); LOESER, David, 72108 Rottenburg (DE); AMANN, Marcus, 72336 Balingen (DE); PAESCH, Markus, 72461 Albstadt (DE); KELES, Yasar, 72393 Burladingen (DE)
(74) Vertreter: Rüger Abel Patentanwälte PartGmbB

(57) **Zusammenfassung**

Das erfindungsgemäße Gewebe- Versiegelungsinstrument (11) weist zwei Branchen (13, 14) auf, die beide jeweils mit Versiegelungselektroden (18, 19, 33, 34) versehen sind. Während eine der Branchen eine Schneidelektrode (25) enthält, weist die andere ein elastisches Widerlager (35) auf. Die Schneidelektrode (25) ist in einem flexiblen Kunststoffkörper (24) gehalten, der in einer schmalen Nut (21) sitzt. Dagegen sitzt das Widerlager (35) in einer vergleichsweise wesentlich breiteren Ausnehmung. Damit ist das Widerlager (35) breiter (vorzugsweise deutlich breiter) als der Isolierkörper (24), der die Schneidelektrode (25) aufweist. Diese Festlegung unterschiedlicher Abmessungen eröffnet einen weiten Spielraum zur Festlegung gewünschter Eigenschaften des Gewebeversiegelungsinstruments (11).

## Beschreibung

Die Erfindung betrifft ein Gewebeversiegelungsinstrument, insbesondere ein zangenartiges Instrument mit Versiegelungselektroden an seinen beiden Branchen und mit einer feststehenden Schneidelektrode.

Gewebeversiegelungsinstrumente der genannten Art sind sowohl für den offenchirurgischen Gebrauch, für den laparoskopischen Gebrauch oder auch für den endoskopischen Einsatz grundsätzlich bekannt und im Markt verfügbar. Beispiele zur Ausbildung solcher Instrumente und insbesondere ihres Kopfs, der im Wesentlichen die beiden Branchen nebst Versiegelungs- und Schneidelektroden umfasst, sind beispielsweise der EP 2 992 849 B1 zu entnehmen. Solche Instrumente weisen zwei Branchen auf, von denen mindestens eine beweglich gelagert ist, um zusammen mit der anderen Branche Gewebe nach Art einer Zange greifen zu können. Während in der einen Branche eine in einem Isolierkörper gehaltene Schneidelektrode angeordnet ist, weist die andere Branche ein Widerlager auf, das selbst elastisch nachgiebig ist und das Gewebe gegen die Schneidelektrode drängt.

Das Instrument dient insbesondere zur Versiegelung und Trennung von Gefäßen, beispielsweise Blutgefäßen. Um die beiden Enden eines durchtrennten Blutgefäßes auch während der noch stattfindenden Versiegelung sicher in dem Instrument zu halten, sind zu beiden Seiten der Schneidelektrode Gewebeaufnahmeräume gebildet, in denen die wulstartigen Enden des durchtrennten Gefäßes formschlüssig gehalten sind, solange das Instrument geschlossen ist.

Ein ähnliches Instrument ist aus der EP 3 138 522 B1 bekannt. Dieses Instrument zeichnet sich durch ein aus Silikon gebildetes flexibles Widerlager aus, das sich beim Gewebeschnitt um die Schneidelektrode faltet.

Weitere Gewebeversiegelungselemente mit Schneidelektrode sind aus der EP 1 632 192 A1**,** der EP 2 409 653 B1**,** der US 8,679,115 B2 und der EP 1 632 192 A1 bekannt.

Insbesondere bei Instrumenten, die durch einen beschränkten Kanal, beispielsweise den Arbeitskanal eines Endoskops oder durch einen Trokar oder dergleichen, in den Körper eines Patienten einzuführen sind, ist die Baubreite des Instrumentes und somit auch seines Versiegelungswerkzeugs beschränkt. Außerdem sollten die Branchen des Instrumentes typischerweise seitlich gekrümmt sein, um die Arbeit an Organen zu erleichtern, die naturgemäß gekrümmte Oberflächen haben.

Unter all diesen Bedingungen soll das Gewebe bei geschlossenem Instrument fest in diesem gefasst sein, um nicht vor Beendigung des Behandlungsvorgangs entkommen zu können.

Davon ausgehend ist es Aufgabe der Erfindung, ein verbessertes Instrument zu schaffen.

Diese Aufgabe wird mit dem Gewebeversiegelungsinstrument nach Anspruch 1 gelöst:

Das erfindungsgemäße Gewebeversiegelungsinstrument weist zwei Branchen auf, die nach Art einer Zange aufeinander zu- und voneinander weg bewegbar sind. In einer der Branchen ist eine Nut vorgesehen, um einen Isolierkörper mit einer darin gefassten Schneidelektrode aufzunehmen. Die andere Branche weist eine ebenfalls nutartige Ausnehmung auf, in der ein flexibles Widerlager für die Schneidelektrode angeordnet ist. Die Besonderheit der Erfindung liegt in dem Umstand, dass die Ausnehmung und mit ihr das Widerlager breiter, vorzugsweise wesentlich breiter, ist als die Breite der Nut. Damit ergibt sich die Möglichkeit, die Schneidelektrode in einem flexiblen Isoliermaterial anzuordnen, sodass die Schneidelektrode seitlich geringfügig federn kann, ohne dabei instabil zu werden. Die breitere Ausnehmung für das Widerlager ermöglicht es, Gewebeaufnahmeräume zu gestalten, die einerseits geräumig sind, andererseits aber insbesondere durch Materialverdrängungseffekte des Widerlagers dazu führen, dass das im Aufnahmeraum gefasste Gewebe unter Druck gesetzt und festgehalten wird.

Vorzugsweise überspannt das elastische Widerlager in seiner Breite nicht nur die Schneidelektrode und den sie haltenden Isolierkörper, sondern zusätzlich den Abstand zwischen dem Isolierkörper und den seitlichen Koagulationselektroden. Die in diesem Abstand vorhandenen Flächen sind vorzugsweise unnachgiebig, sodass sie die Position des Gewebes relativ zu der Schneidelektrode eindeutig festlegen. Dies kann sowohl die Qualität des Schnitts fördern, als auch die Sicherheit der Verankerung der wulstigen Gewebeenden in den Gewebeaufnahmeräumen. Auch wird mit diesem Design die Stabilität der die Schneidelektrode aufnehmenden Branche gefördert.

Die Versiegelungselektroden sind an den beiden Branchen jeweils vorzugsweise randständig angeordnet. Sie folgen dann der äußeren Kontur der Branche. Dadurch wird die laterale Ausdehnung der Gewebeaufnahmeräume maximiert und der zur Verfügung stehende Raum innerhalb des Instruments bestmöglich genutzt.

Die Versiegelungselektroden der Branche erstrecken sich jeweils vorzugsweise von einem scharnierseitigen Endbereich ausgehend zu einem distalen Ende der Branche hin. Die Versiegelungselektroden jeder Branche können an ihren distalen Enden untereinander verbunden sein. Insbesondere können die Versiegelungselektroden jeweils von einem einzigen, nahtlos zusammenhängenden, im Wesentlichen u-förmigen Bauteil gebildet sein. Dabei ist es vorteilhaft, wenn eine der Versiegelungselektroden jeder Branche sich von einem gelenknahen Bereich zu einem distalen Endbereich zunächst entlang einer Geraden erstreckt, woran sich ein Abschnitt mit geringem Radius anschließt. Hingegen erstreckt sich die andere der Versiegelungselektroden von dem gelenknahen Bereich zu dem distalen Endbereich hin in einem Bogen mit einem vergleichsweise (viel) größeren Radius. Dieser Bogen ist vorzugsweise so angeordnet, dass die Versiegelungselektroden an einer Stelle ihren minimalen Abstand zueinander haben, die zwischen dem gelenknahen Bereich und dem distalen Endbereich oder an diesen angrenzend liegt. Auf diese Weise wird insbesondere sowohl im gelenknahen Endbereich wie auch im distalen Endbereich eine solche Breite des zwischen den Versiegelungselektroden eingeschlossenen Raums geschaffen, dass biologisches Gewebe darin besonders sicher gefasst werden kann. Die sichere Fassung des Gewebes im gelenknahen Bereich und im Endbereich ist besonders wichtig, um ein ungewolltes Herausziehen der Gewebeenden aus dem geschlossenen Werkzeug zu verhindern. Auch fördert diese Konfiguration die Gleichmäßigkeit des am Gewebe erzielten Effekt über die Länge der Schneidelektrode und der Versiegelungselektroden. Diese Form der Versiegelungselektroden ist unabhängig von der Breite der Nut und der Breite der Ausnehmung sowie deren Übereinstimmung oder Nichtübereinstimmung vorteilhaft. Außerdem ist diese Form der Versiegelungselektroden in Verbindung mit der nachfolgend im Zusammenhang mit erfindungsgemäßen Ausführungsformen beschriebenen Form der Schneidelektrode vorteilhaft. Dies wiederum unabhängig von der Breite der Nut, der Breite der Ausnehmung, der Form der Versiegelungselektroden sowie deren Übereinstimmung oder Nichtübereinstimmung.

Vorzugsweise folgt die Ausnehmung für das Widerlager der Form der Versiegelungselektroden der zweiten Branche. Hingegen folgt die Nut der ersten Branche der Form der Versiegelungselektroden nicht. Vielmehr ist die Nut vorzugsweise parallelflankig ausgebildet, sodass die Nutflanken zu den benachbarten Versiegelungselektroden wechselnde Abstände aufweisen. Dies eröffnet die Möglichkeit der einfachen Fertigung insbesondere hinsichtlich der Erzeugung der Nut und Gleichzeitig die Möglichkeit der davon unabhängigen Gestaltung der Form der Versiegelungselektroden. Die Versiegelungselektrode kann dabei eine einfache bogenförmige Krümmung oder auch eine doppelte Krümmung (leichte S-Form) aufweisen. Dieses Merkmal kann auch bei einem Werkzeug vorgesehen sein, bei dem die Ausnehmung die gleiche Breite aufweist, wie die Nut, wobei das Werkzeug ansonsten mit dem beschriebenen erfindungsgemäßen Werkzeug übereinstimmt. In diesem Fall fluchten die Ausnehmung und die Nut miteinander, so dass das Widerlager und die Schneidelektrode beim Schließen genau übereinander kommen. Jedoch gilt auch hier, dass die Nut entlang ihrer Länge wechselnde Abstände zu den Versiegelungselektroden haben kann. Zum Beispiel kann die Nut oder die Schneidelektrode an einer ungefähr mittigen Stelle ihrer Längserstreckung einen minimalen Abstand zu der einen und einen maximalen Abstand zu der anderen Versieglungselektrode aufweisen. Außerdem können die Abstände der Nut zu den beiden Versiegelungselektroden entlang ihrer Längserstreckung auf unterschiedliche Weise variieren. Z.B. kann der Abstand zu einer der Versiegelungselektroden lokal zunehmen, während er an der gleichen Stelle zu der anderen Versiegelungselektrode lokal abnimmt.

Vorzugsweise ist der Isolierkörper flexibel ausgebildet, wobei er einen in der Nut gefassten ersten Abschnitt und einen aus der Nut herausragenden zweiten Abschnitt aufweist. Die Schneidelektrode erstreckt sich dabei durch den zweiten Abschnitt hindurch in den ersten Abschnitt hinein, wodurch auch bei sehr schlanker Ausbildung des Isolierkörpers eine gute seitliche Stabilität der Schneidelektrode erhalten und die elektrische Isolation sicher gestellt wird. Die Schneidelektrode erstreckt sich dabei in die Nut, so dass der Teil der Schneidelektrode, der eine freiliegende Schneidkante aufweist, oberhalb der Nut und ein Teil der Schneidelektrode innerhalb der Nut steht. Der aus der Nut herausstehende Teil der Schneidelektrode kann an seinen beiden Flachseiten von dem Isoliermaterial des Isolierkörpers bedeckt und somit elektrisch isoliert sein. Dabei kann die Nut zur Aufnahme des Isolierkörpers von dem gelenknahen Bereich zu dem distalen Bereich hin bogenförmig (einfach gekrümmter Bogen) oder auch s-förmig (doppelt gekrümmter Bogen) verlaufen. Bedarfsweise kann die Nut aber auch in einem Abschnitt, insbesondere ihrem distalen Ende mit aufeinander zu verlaufenden Nutwänden versehen sein.

Weiter ist es vorteilhaft, wenn das Widerlager einen Abschnitt aufweist, mit dem es sich an dem Boden der Nut vorzugsweise etwa mittig abstützt. In Verbindung mit optional vorsehbaren Leerräumen zwischen der Branche und dem Widerlager können die Nachgiebigkeit des Widerlagers und die Kraft (insbesondere der wegabhängige Verlauf der Kraft), mit der das Widerlager beidseits der Schneidelektrode auf das Gewebe drückt, zweckdienlich eingestellt werden. Insbesondere kann eine hohe anfängliche Kraft schon bei geringer Verformung des Widerlagers erreicht werden. Dabei ist es ergänzend hinsichtlich der Fertigung und der Sicherung des Widerlagers in der Branche vorteilhaft, wenn das Widerlager unmittelbar an die Versiegelungselektroden anschließt und sie somit gegen gefasstes biologisches Gewebe isoliert. Auch werden durch den direkten Anschluss des Widerlagers an die inneren Flanken der Versiegelungselektrode und die sich daraus ergebende elektrische Isolation der Flanken Kriechströme wirksam unterbunden. Durch die Abstützung des Widerlagers an dem Boden der Ausnehmung wird die Verbindung zwischen dem Widerlager und der Versiegelungselektrode von Kräften freigehalten, sodass eine dort ausgebildete Haft- oder Klebeverbindung nicht wesentlich beeinträchtigt wird. Unmittelbar im Anschluss des Widerlagers an die Versiegelungselektrode kann ein Abstand zwischen dem Widerlager und der Branche ausgebildet sein. Dieser Abstand bewirkt eine kräftemäßige Entkopplung des Widerlagers von dem Anschluss an die Versiegelungselektrode und trägt somit zur Dauerhaftigkeit der stoffschlüssigen Verbindung an dieser Stelle bei.

Weitere Einzelheiten vorteilhafter Ausführungsformen der Erfindung sind Gegenstand der Beschreibung oder von Unteransprüchen sowie der ergänzenden Zeichnung. In dieser zeigen:
Figur 1 das erfindungsgemäße Instrument mit seinem Werkzeugteil, in ausschnittsweiser perspektivischer Darstellung.
Figur 2 und 3 die beiden Branchen des Instruments nach Figur 1, jeweils in Draufsicht.
Figur 4 die Branchen des Instruments nach Figur 1, in einer Querschnittsansicht an der Stelle A₂₈.
Figur 5 und 6 weitere Ausführungsformen der Branchen des erfindungsgemäßen Instruments, in Querschnittsdarstellung an der Stelle A₂₈.
Figur 7 die Branchen nach Figur 4 beim Versiegeln und Trennen eines Gefäßes.
Figur 8 die Branchen nach Figur 5 beim Versiegeln und Trennen eines biologischen Gefäßes und
Figur 9 die Branchen nach Figur 6 beim Versiegeln und Trennen eines Gefäßes.

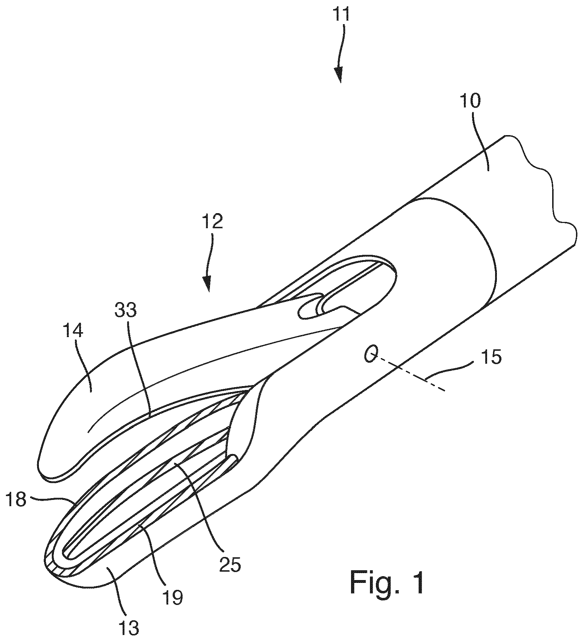

In Figur 1 ist das distal an einem Schaft 10, eines Versiegelungsinstruments 11, angebrachte Werkzeug 12, veranschaulicht. Zu diesem gehören eine erste Branche 13 und eine zweite Branche 14, die nach Art einer Zange aufeinander zu- und voneinander weg schwenkbar angeordnet sind. Dazu ist mindestens eine der beiden Branchen 13 und 14, im vorliegenden Ausführungsbeispiel die zweite Branche 14, mittels einer durch ihre Schwenkachse 15 angedeutete Scharniereinrichtung gegen die erste Branche 13 schwenkbar, die feststehend angeordnet ist. Alternativ können auch beide Branchen 13 und 14 aufeinander zu- und voneinander wegschwenkbar angeordnet sein. Die Scharniereinrichtung kann durch ein oder zwei Schwenklager, eine Kulissenführung, ein Federgelenk oder dergleichen gebildet sein.

Das Gewebeversiegelungsinstrument 11 dient insbesondere zum Schließen, Versiegeln und Trennen von Gefäßen, beispielsweise Blutgefäßen, kann aber auch für andere operative Tätigkeiten, beispielsweise das Präparieren von Organen oder anderen biologischen Geweben eingesetzt werden.

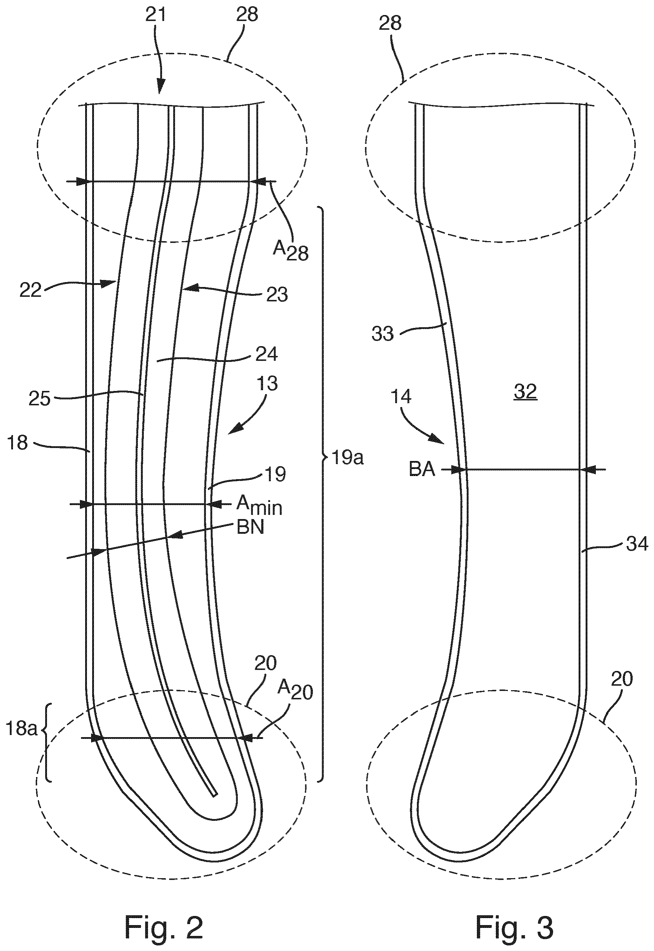

Zur Erläuterung des weiteren Aufbaus der Branchen 13 und 14 wird auf die Figuren 2, 3 und 4 verwiesen. Die erste Branche 13 ist durch ein steifes, beispielsweise aus Metall ausgebildetes Tragteil 16 gebildet, das an seiner Außenseite 17 eine elektrische Isolation tragen kann. Alternativ kann das Tragteil 16 auch teilweise oder auch komplett aus einem mechanisch stabilen, wenig oder nicht flexiblen und elektrisch isolierenden Kunststoff bestehen. Auch kann das Tragteil 16 ein Verbundteil sein und z.B. aus einem kunststoffumspritzten Metall-Inlay gebildet sein.

Entlang seiner beiden Ränder ist das Tragteil 16 der Branche 13 mit Versiegelungselektroden 18 und 19 versehen, die über nicht weiter dargestellte Leitungen mit einem elektrischen Generator verbindbar sind. Die beiden Versiegelungselektroden 18 und 19 können in einem distalen Endbereich 20 der Branche 13 untereinander körperlich und elektrisch verbunden sein, wie es in Figur 2 dargestellt ist. Alternativ können jedoch auch gesonderte Versiegelungselektroden 18 und 19 vorgesehen sein, die auf gleichen oder unterschiedlichen Potenzialen liegen und in dem distalen Endbereich 20 körperlich nicht verbunden sind.

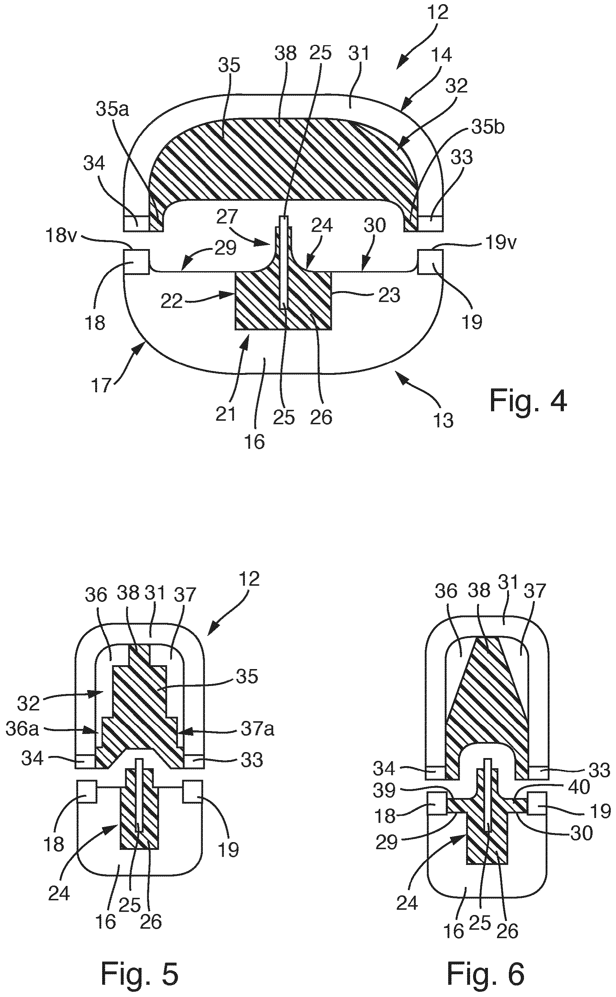

Die erste Branche 13 weist zwischen den Versiegelungselektroden 18 und 19 eine Nut 21 auf, die von Nutflanken 22, 23 begrenzt ist. Die Nutflanken 22, 23 sind vorzugsweise, wie aus den Figuren 2 und 4 hervorgeht, in konstantem Abstand und somit parallel zueinander angeordnet, wobei ihr Abstand die Nutbreite BN, festlegt. Vorzugsweise variiert die Nutbreite BN über die Länge der Nut 21 nicht oder nur in einem Abschnitt, vorzugsweise in einem distalen Endabschnitt derselben. Die Nut 21 weist vorzugsweise einen Rechteck- oder Quadratquerschnitt auf. Es ist aber auch möglich, der Nut 21 einen Trapezquerschnitt zu verleihen, so dass sie am Boden enger ist als an ihrer der anderen Branche 14 zugewandten Mündung.

In der Nut 21 ist ein Isolierkörper 24 angeordnet, der beispielsweise aus Silikon oder aber auch aus einem anderen isolierenden Stoff, insbesondere Kunststoff, ausgebildet sein kann. Vorzugsweise findet ein Kunststoff Anwendung, der eine spürbare Flexibilität oder Elastizität sowie eine hohe Kriechstromfestigkeit aufweist. Dies kommt sowohl der mechanischen als auch der elektrischen Funktion des Gewebeversiegelungsinstruments 11 erheblich zugute.

In dem Isolierkörper 24 ist eine Schneidelektrode 25 angeordnet, die durch einen dünnen Blechstreifen gebildet ist. Dieser kann eine oder mehrere Öffnungen aufweisen, die von dem Isolierkörper 24 durchsetzt sind, um die Schneidelektrode 25 sicher in dem Isolierkörper 24 zu verankern. Die Schneidelektrode 25 ist an ihren beiden Flanken von dem Isolierkörper 24 bedeckt und somit elektrisch isoliert. Der Isolierkörper bildet somit eine in Richtung der Branche 14 aufragende Wand, an der oben die Schneidelektrode 25 frei liegt. Es liegt dort lediglich eine schmale Stirnkante der Schneidelektrode 25 sowie gegebenenfalls ein schmaler streifenförmiger an diese anschließender seitlicher Bereich frei. Dieser seitliche Bereich ist vorzugsweise nicht breiter, als die Stirnkante breit ist.

Der Isolierkörper 24 weist einen in der Nut 21 sitzenden ersten Abschnitt 26 und einen sich von diesem weg erstreckenden, eine aufragende Wand bildenden zweiten Abschnitt 27 auf. Die Schneidelektrode 25 erstreckt sich durch den zweiten Abschnitt 27 hindurch bis in den ersten Abschnitt 26 hinein.

Zur Form der ersten Branche 13 in Längsrichtung wird auf Figur 2 verwiesen. Von einem scharniernahen Bereich 28 zu dem distalen Endbereich 20 erstreckt sich die Versiegelungselektrode 18 entlang einer weitgehend geraden Linie. Daran schließt sich in dem Endbereich 20 oder in Nachbarschaft desselben ein Bogenbereich 18a mit großer Krümmung an. Hingegen verläuft die Versiegelungselektrode 19 ausgehend von dem scharniernahen Bereich 28 in einem Abschnitt 19a in einem Bogen, mit geringer Krümmung. Diese Krümmung ist geringer als die Krümmung in dem Bereich 18a. Im Verlauf des Bereichs 19a nähert sich die Versiegelungselektrode 19 der Versiegelungselektrode 18 an und entfernt sich wieder von dieser. Beispielsweise kann der minimale Abstand Aₘᵢₙ zwischen den Versiegelungselektroden 18 und 19, wie in Figur 2 dargestellt, zwischen dem scharniernahen Bereich 28 und dem distalen Endbereich 20 oder auch nahe bei oder in dem distalen Endbereich 20 liegen. Somit sind der Abstand A₂₈ im scharniernahen Bereich 28 und der Abstand A₂₀ im distalen Endbereich 20 vorzugsweise größer als der dazwischen gemessene Abstand Aₘᵢₙ. Die Abstände A₂₀, Aₘᵢₙ und A₂₈ sind dabei stets quer (rechtwinklig) zu der linear gestreckten Versiegelungselektrode zu messen. Die Messrichtung stimmt mit der Richtung überein, in der Gewebezugkräfte vorwiegend wirken. In dem distalen Endbereich 20 nähern sich die Versiegelungselektroden 18 und 19 an, um an dem distalen Ende ineinander überzugehen. Die Größe des scharniernahen Bereichs 28 und des distalen Endbereichs 20 kann verschieden festgelegt sein. Z.B. kann der scharniernahe Endbereich enden, wo der lineare Abschnitt der Versiegelungselektrode 19 endet. Der distale Endbereich 20 kann beginnen, wo der lineare Abschnitt der Versiegelungselektrode 18 endet. Unabhängig davon liegt der Bereich mit dem Abstand Aₘᵢₙ jedenfalls zwischen die Bereichen 20, 28.

Die Nut 21 weist einen anderen Verlauf auf. Die Nut 21 kann einem hinsichtlich seiner Krümmung minimierten Bogen folgen, im Verlauf dessen sie sich der geraden Versiegelungselektrode 18 etwa mittig zwischen dem scharniernahen Bereich 28 und dem distalen Endbereich 20 etwas annähert, ohne den minimalen Isolationsabstand zu unterschreiten. Sie kann dabei einem einfachen Bogen folgen oder auch leicht S-förmig gekrümmt sein, was sich daraus ergeben kann, dass die Schneideelektrode 25 im scharniernahen Bereich 28 wenigstens abschnittsweise parallel zu der Versiegelungselektrode 18 angeordnet ist. Charakteristisch bei zumindest den meisten Ausführungsformen der Erfindung ist, dass die Schneidelektrode 25 zu zumindest einer der Versiegelungselektroden 18, 19, hier zu der Versiegelungselektrode 18, einen wechselnden Abstand aufweist. Dieser ist beginnend in dem scharniernahen Bereich zunächst groß. Weiter von dem scharniernahen Bereich 20 weg, etwa mittig der Branche 13 ist der Abstand minimal und im distalen Endbereich 20 ist er wieder etwas größer. Diese Elektrodenkonfiguration kann auch bei Werkzeugen angewendet werden, bei denen die Nut 21 und die Ausnehmung 32 übereinstimmende Breiten und Formen aufweisen. Für solche Werkzeuge gilt die übrige Beschreibung entsprechend.

Wie Figur 4 erkennen lässt, sind zu beiden Seiten des Isolierkörpers 24 Flächenbereiche 29, 30 der ersten Branche 13 vorgesehen, die sich einerseits an den Isolierkörper 24 und andererseits an die Versiegelungselektroden 18 und 19 anschließen. Diese Flächenbereiche 29 und 30 können metallisch blank sein und in elektrischem Kontakt mit den Versiegelungselektroden 18, 19 stehen. Es wird jedoch bevorzugt, diese Flächenbereiche 29, 30 mit einer isolierenden Beschichtung oder Auflage zu versehen, beispielsweise einer Keramikauflage, einer Kunststoffauflage (z.B. aus Parylene) oder dergleichen. Auch kann das Tragteil 16 aus Kunststoff (mit oder ohne Metallinlay) ausgebildet sei, so dass eine gesonderte Isolation entbehrlich ist. Die Flächenbereiche 29, 30 können gegenüber den an den Elektroden 18, 19 vorgesehenen Versiegelungsflächen 18v, 19v zurückgesetzt, d.h. in Figur 4 tiefergesetzt sein. Außerdem können die Flächenbereiche stufenlos an den Isolierkörper 24 anschließen.

Die in Figur 4 dargestellten Versiegelungsflächen 18v, 19v der Versiegelungselektroden 18, 19 sind "schmal" ausgebildet. Dies bedeutet, dass ihre in Figur 4 in der Zeichenebene zu messende Breite geringer, vorzugsweise deutlich geringer ist, als der in gleicher Richtung zu messende Abstand zwischen der Versiegelungselektrode 18 und der Schneidelektrode 25. Gleiches gilt für die Versiegelungselektrode 19. Diese Bedingung gilt zumindest für einen Abschnitt der Versiegelungselektrode 18, 19 oder vorzugsweise für die gesamte Länge derselben. Diese Gestaltung führt zu einem schmalen aber verlässlichen Versiegelungssaum an dem Gewebe und schafft außerdem einen großen Gewebeaufnahmeraum, in dem das Gewebe während der Behandlung gesichert ist.

Die zweite Branche 14 weist wiederum ein Tragteil 31 auf, das eine nutartige Ausnehmung 32 (Figur 3) begrenzt. Das Tragteil 31 kann wiederum aus Metall oder auch aus Kunststoff oder einem Metall-Kunststoffverbundteil gebildet sein. An seinem äußeren Rand trägt es Versiegelungselektroden 33, 34, die sich von dem scharniernahen Bereich 28 bis zu dem distalen Endbereich 20 erstrecken. Die der Kontur des Tragteils 31 folgenden Versiegelungselektroden 33, 34 sind spiegelsymmetrisch zu den Versiegelungselektroden 18, 19 angeordnet. Sind die Branchen geschlossen, deckt sich die Versiegelungselektrode 34 mit der Versiegelungselektrode 18. Außerdem deckt sich die Versiegelungselektrode 33 mit der Versiegelungselektrode 19. Insoweit gilt die vorstehende Beschreibung des Verlaufs und der Form der Versiegelungselektroden 18, 19 entsprechend spiegelsymmetrisch für die Versiegelungselektroden 33, 34. Außerdem können die Versiegelungselektroden 33, 34 in dem distalen Endbereich 20 untereinander elektrisch und körperlich verbunden sein.

Die Ausnehmung 32 weist eine Breite BA auf, die entlang der Längserstreckung der zweiten Branche 14 variieren kann. An jedem Punkt ist sie jedoch größer als die Breite BN der Nut 21. Die Breite BA stimmt vorzugsweise mit dem Abstand zwischen den Versiegelungselektroden 33, 34 überein. Somit sind die Versiegelungselektroden 33, 34 und die Ausnehmung 32 konturgleich. Es wird aber darauf hingewiesen, dass die Kontur der Versiegelungselektroden 33, 34 und der Ausnehmung 32 auch unterschiedlich festgelegt sein können. Der Abstand zwischen den Versiegelungselektroden 33, 34 und sein Verlauf entlang der Länge der Branche 14 stimmt mit dem Abstand und dem Verlauf der Versiegelungselektroden 18, 19 überein - diese sind weitgehend deckungsgleich.

In der Ausnehmung 32 ist ein aus flexiblem Kunststoff bestehendes Widerlager 35 angeordnet. Dieses besteht vorzugsweise aus einem flexiblen Kunststoff, beispielsweise Silikon. Vorzugsweise ist das Widerlager ein geschlossener einteiliger Körper, der frei von Hohlräumen ist. Das Widerlager 35 ist verformbar, jedoch nicht relevant komprimierbar. Dies bedeutet, dass sich in der bevorzugten Ausführungsform sein Volumen durch die im Werkzeug wirkenden Klemmkräfte nicht relevant reduzieren lässt.

Das Widerlager 35 kann die Ausnehmung 32 vollständig ausfüllen oder, wie es Figur 4 andeutet, kleinere Lufttaschen frei lassen, sodass das Widerlager 35 bei Verformung eine der Größe der Lufttaschen entsprechende Ausweichbewegung ausführen kann. Außerdem erstreckt sich der Widerlagerkörper 35 mit Fortsätzen 35a, 35b an die Innenseiten der Versiegelungselektroden 33, 34, an denen er angebracht ist. Die Versiegelungselektroden 33, 34 sind dadurch zu den Gewebeaufnahmeräumen hin elektrisch isoliert. Durch die Abstützung des Abschnitts 38 des Widerlagers 35 an dem Boden der Ausnehmung 32 sind die Verbindungsstellen zwischen den Fortsätzen 35a, 35b und den Innenseiten der Versiegelungselektroden 33, 34 weitgehend kräftefrei, so dass die bestehende Verbindung nicht bzw. kaum belastet oder gar beeinträchtigt wird.

An der insoweit beschriebenen Ausführungsform des Werkzeugs 12 sind zahlreiche Abwandlungen möglich. Zum Beispiel kann das Werkzeug 12 gemäß Figur 5 deutlich schmaler ausgebildet sein. Optional vorsehbare Lufttaschen zwischen dem Tragteil 31 und dem Widerlager 35 können dazu genutzt werden, die Verformung des Widerlagers in Gebrauch gezielt zu steuern. Jedenfalls aber weist das Widerlager 35, wie schon bei dem Ausführungsbeispiel nach Figur 4, einen Abschnitt 38 auf, der sich direkt an dem Boden der Ausnehmung 32 abstützt. Die Lufttaschen 36, 37 können sich außerdem, wie Figur 5 bespielhaft zeigt, mit Abschnitten 36a, 37a bis an die Verbindungsstellen zwischen dem Widerlager 35 und den Versiegelungselektroden 33, 34 erstrecken. Dadurch können die Verbindungsstellen von mechanischen Scher- und Zugkräften entlastet werden.

Auch bei der Ausführungsform nach Figur 6 können Lufttaschen 36, 37 (nicht dargestellt) und der abstützende Abschnitt 38 vorgesehen sein, weswegen die vorige Beschreibung unter Zugrundelegung gleicher Bezugszeichen entsprechend gilt. Eine Besonderheit der Ausführungsform nach Figur 6 liegt in dem Isolierkörper 24, der sich über die Flächenbereiche 29, 30 erstreckende Fortsätze 39, 40 aufweist. Ansonsten gilt die übrige Beschreibung der vorigen Ausführungsbeispiele entsprechend.

Bei allen Ausführungsformen können die Versiegelungselektroden 18 19 in eine entsprechende Ausnehmung des Stützteils 16 eingesetzt sein. Dies erleichtert die Montage und ermöglicht eine präzise Positionierung der Versiegelungselektroden 18, 19.

Weiter ist es möglich, die Seitenwände 22, 23 der Nut 21 mit einer oder mit mehreren seitlichen Ausbuchtungen zu versehen, in die sich der Isolierkörper 24 mit entsprechenden Vorsprüngen hinein erstreckt. Die Ausnehmungen können entlang nur einer der beiden Seitenwände oder auch entlang beider Seitenwände 22, 23 angeordnet sein. Dies gestattet die gezielte Festlegung der seitlichen Flexibilität der Elektrode 25. Die Elektrode 25 kann an ihrer in der Nut 21 liegenden Kante auch seitliche Fortsätze aufweisen. Diese können sich zusammen mit dem Isolierkörper 24 in die seitlichen Ausnehmungen hinein erstrecken, sofern vorhanden. Diese Maßnahme ermöglicht eine besonders gute seitliche Stabilisierung der Schneidelektrode bei zugleich Verwendung eines hoch flexiblen Kunststoffs für den Isolierkörper 24.

Das insoweit beschriebene Gewebeversiegelungsinstrument 11 arbeitet wie folgt:

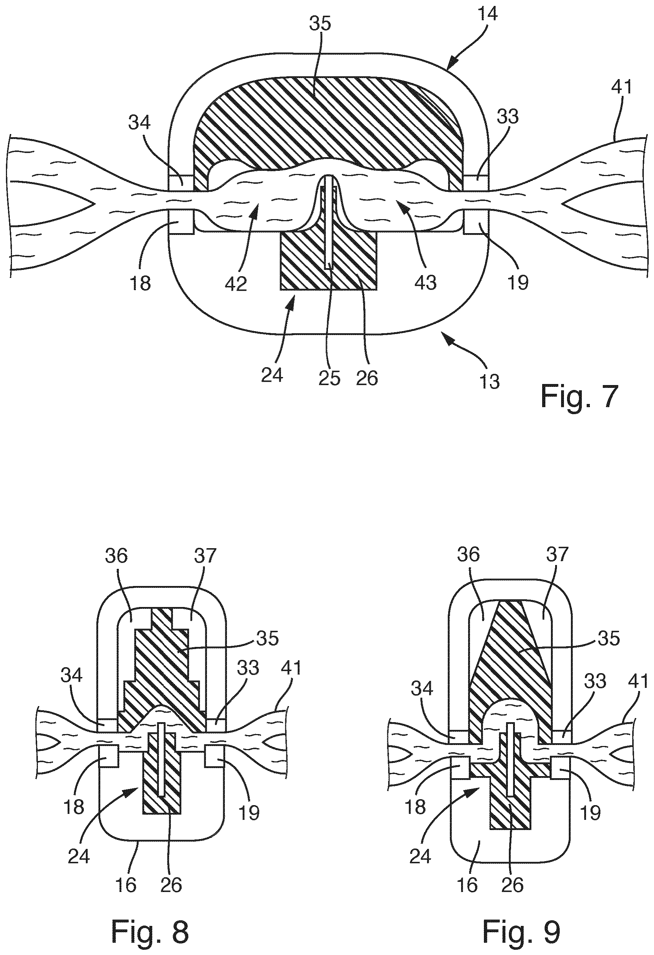

In Gebrauch wird Gewebe zwischen den Branchen 13, 14 gefasst, wie es beispielsweise in Figur 7 veranschaulicht ist. Die Versiegelungselektroden 33, 34 sind dabei an einen Pol eines elektrischen Generators angeschlossen, während die Versiegelungselektroden 18, 19 an einen anderen Pol des Generators angeschlossen sind. Das zwischen den Branchen 13, 14 gefasste und zusammengedrückte biologische Gewebe, beispielsweise das Gefäß 41, wird deswegen zwischen den Versiegelungselektroden 18, 33; 19, 34 fusioniert und koaguliert. Dabei drückt die Schneidelektrode 25 das biologische Gewebe gegen das Widerlager 35, das dem Druck ausweichen kann. Im Gegenzug drückt es jedoch in einer Ausweichbewegung zu beiden Seiten der Schneidelektrode 25 gegen das Gewebe. Dieses ist in den Gewebeaufnahmeräumen 42, 43 gefasst, die zu beiden Seiten der Schneidelektrode 25 gebildet sind. Durch die Ausweichbewegung des Widerlagers 35, d.h. das Zurückweichen vor dem Gewebe 41 wird in den in den Aufnahmeräumen 42, 43 Platz für das Gewebe 41 geschaffen. So werden die Enden des Gewebes 41, beispielsweise Gefäßes, gut in dem Instrument festgehalten und sind davor sicher, ungewollt zu entkommen. Zeitgleich zu der Gewebeversiegelung zwischen den Elektrodenpaaren 18/34, 33/19 oder zeitlich versetzt wird die Schneidelektrode 25 bestromt, so dass diese das Gewebe 41 durchtrennt.

Bei schmaleren Instrumenten, wie sie in Figur 8 und 9 dargestellt sind, sind die Verhältnisse ähnlich. Jedoch kann dort durch die Lufttaschen 36, 37 ein Ausweichen des Widerlagers 35 auch in die Lufttaschen hinein ermöglicht werden, um den Druck auf das Gewebe 41 nicht übermäßig ansteigen zu lassen.

Das erfindungsgemäße Gewebe- Versiegelungsinstrument 11 weist zwei Branchen 13, 14 auf, die beide jeweils mit Versiegelungselektroden 18, 19, 33, 34 versehen sind. Während eine der Branchen eine Schneidelektrode 25 enthält, weist die andere ein elastisches Widerlager 35 auf. Die Schneidelektrode 25 ist in einem flexiblen Kunststoffkörper 24 gehalten, der in einer schmalen Nut 21 sitzt. Dagegen sitzt das Widerlager 35 in einer vergleichsweise wesentlich breiteren Ausnehmung. Damit ist das Widerlager 35 breiter (vorzugsweise deutlich breiter) als der Isolierkörper 24, der die Schneidelektrode 25 aufweist. Diese Festlegung unterschiedlicher Abmessungen eröffnet einen weiten Spielraum zur Festlegung gewünschter Eigenschaften des Gewebeversiegelungsinstruments 11.

### Bezugszeichen:

- 10: Schaft
- 11: Gewebeversiegelungsinstrument
- 12: Werkzeug
- 13: erste Branche
- 14: zweite Branche
- 15: Lagereinrichtung, Gelenkachse
- 16: Tragteil
- 17: Außenseite der ersten Branche 13
- 18, 19: Versiegelungselektroden
- 18v, 19v: Versiegelungsflächen
- 18a: Abschnitt der Versiegelungselektrode 18
- 19a: Abschnitt der Versiegelungselektrode 19
- 20: distaler Endbereich
- 21: Nut
- 22, 23: Nutflanken
- 24: Isolierkörper
- 25: Schneidelektrode
- 26: erster Abschnitt (Fußabschnitt) des Isolierkörpers
- 27: zweiter Abschnitt (Wandabschnitt) Des Isolierkörpers
- 28: scharniernaher Bereich
- 29, 30: Flächenbereiche
- 31: Tragteil
- 32: Ausnehmung
- 33, 34: Versiegelungselektroden
- 35: Widerlager
- 35a, 35b: Fortsätze des Widerlagers 35
- 36, 37: Lufttaschen
- 38: Abschnitt des Widerlagers
- 39, 40: Abschnitte des Isolierkörpers
- 41: Gewebe
- 42, 43: Aufnahmeräume

## Patentansprüche

1. Gewebeversiegelungsinstrument (11) mit einer ersten Branche (13) und einer zweiten Branche (14), die aufeinander zu und voneinander weg mittels einer Lagereinrichtung (15) schwenkbar gelagert sind und die jeweils Versiegelungselektroden (18, 19; 33, 34) aufweisen,
wobei die erste Branche (13) zwischen den Versiegelungselektroden (18, 19) eine Nut (21) aufweist, die von zwei einander gegenüberliegenden Flanken (22, 23) begrenzt ist, deren Abstand zueinander eine erste Breite (BN) festlegt, wobei in der Nut (21) eine in einem Isolierkörper (24) gelagerte Schneidelektrode (25) gehalten ist, und
wobei die zweite Branche (14) zwischen den Elektroden (33, 34) eine Ausnehmung (32) aufweist, die von zwei einander gegenüberliegenden Seitenflächen begrenzt ist und in der ein flexibles Widerlager (35) für die Schneidelektrode (25) angeordnet ist, wobei der Abstand der Seitenflächen zueinander eine zweite Breite (BA) festlegt,
wobei die erste Breite (BN) geringer ist, als die zweite Breite (BA).

2. Instrument nach Anspruch 1, **dadurch gekennzeichnet, dass** die Versiegelungselektroden (18, 19) der ersten Branche (13) an dieser randständig angeordnet sind.

3. Instrument nach Anspruch 1 oder 2, **dadurch gekennzeichnet, dass** die Versiegelungselektroden (33, 34) der zweiten Branche (14) an dieser randständig angeordnet sind.

4. Instrument nach einem der vorstehenden Ansprüche, **dadurch gekennzeichnet, dass** die Versiegelungselektroden (18, 19) der ersten Branche (13) sich beidseits der Nut (21) zu einem distalen Endbereich (20) hin erstreckend angeordnet sind.

5. Instrument nach Anspruch 4, **dadurch gekennzeichnet, dass** die Versiegelungselektroden (18, 19) der ersten Branche (13) in dem distalen Endbereich (20) untereinander verbunden sind.

6. Instrument nach einem der vorstehenden Ansprüche, **dadurch gekennzeichnet, dass** die Versiegelungselektroden (33, 34) der zweiten Branche (14) sich beidseits der Ausnehmung (32) zu einem distalen Endbereich (20) hin erstreckend angeordnet sind.

7. Instrument nach Anspruch 6, **dadurch gekennzeichnet, dass** die Versiegelungselektroden (33, 34) der zweiten Branche (14) an dem distalen Endbereich (20) untereinander verbunden sind.

8. Instrument nach einem der vorstehenden Ansprüche, **dadurch gekennzeichnet, dass** eine der Versiegelungselektroden (18; 34) jeder Branche (13, 14) sich von einem gelenknahen Bereich (28) zu einem distalen Endbereich (20) entlang einer Geraden erstreckt, während sich die andere der Versiegelungselektroden (19, 33) von dem gelenknahen Bereich (28) zu dem distalen Endbereich (20) in einem Bogen erstreckt, der so angeordnet ist, dass ein minimaler Abstand (Aₘᵢₙ) der Versiegelungselektroden (18, 19; 33, 34) voneinander an einer Stelle zwischen dem gelenknahen Bereich (28) und dem distalen Endbereich (20) oder an den distalen Endbereich (20) grenzend festgelegt ist.

9. Instrument nach einem der vorstehenden Ansprüche, **dadurch gekennzeichnet, dass** die Nut (21) einen bogenförmig gekrümmten Verlauf aufweist.

10. Instrument nach einem der vorstehenden Ansprüche, **dadurch gekennzeichnet, dass** die erste Breite (BN) entlang der Länge der Nut (21) konstant ist.

11. Instrument nach einem der vorstehenden Ansprüche, **dadurch gekennzeichnet, dass** die zweite Breite (BN) entlang der Länge der Ausnehmung (32) variierend festgelegt ist.

12. Instrument nach einem der vorstehenden Ansprüche, **dadurch gekennzeichnet, dass** der Isolierkörper (24) flexibel ausgebildet ist und einen in der Nut (21) gefassten ersten Abschnitt (26) und einen aus der Nut (21) herausragenden zweiten Abschnitt (27) aufweist.

13. Instrument nach Anspruch 12, **dadurch gekennzeichnet, dass** die Schneidelektrode (25) sich durch den zweiten Abschnitt (27) in den ersten Abschnitt (26) hinein erstreckend angeordnet ist.

14. Instrument nach einem der vorstehenden Ansprüche, **dadurch gekennzeichnet, dass** die Ausnehmung einen Boden aufweist und dass das Widerlager (35) einen sich an dem Boden der Ausnehmung (32) abstützenden Abschnitt (38) aufweist.

15. Instrument nach einem der vorstehenden Ansprüche, **dadurch gekennzeichnet, dass** das Widerlager (35) einen an die Versiegelungselektrode (33, 34) anschließenden Abschnitt (35a, 36b) aufweist.
